# EUROPEAN PATENT SPECIFICATION

(11) **EP 2 321 010 B1**
(45) Date of publication and mention of the grant of the patent: **26.12.2012**
(21) Application number: 09805566.8
(22) Date of filing: 06.08.2009
(51) Int. Cl.: A61N 7/02, B25J 11/00, A61B 10/02

(54) **ROBOTIC LOCALIZING AID FOR HIGH INTENSITY FOCUSED ULTRASOUND DELIVERY**
ROBOTERORTUNGSHILFE FÜR HOCHINTENSIVE FOKUSSIERTE ULTRSCHALLABGABE
AIDE ROBOTIQUE À LA LOCALISATION POUR L'ADMINISTRATION D'ULTRASONS FOCALISÉS DE HAUTE INTENSITÉ

(30) Priority: 07.08.2008 US 87087 P
(43) Date of publication of application: 18.05.2011
(73) Proprietor: University Of Rochester, Rochester, NY 14642 (US)
(72) Inventor: DOGRA, Vikram, Pittsford NY 14534 (US); NG, Wan, Singapore 678948 (SG)
(74) Representative: Gillard, Matthew Paul
(86) International application number: PCT/US2009/053011
(87) International publication number: WO 2010/017396

(56) References cited:
- WO-A1-99/22652
- US-A- 5 184 601
- US-A1- 2007 010 805
- US-A1- 2007 088 346
- US-A1- 2007 233 185
- US-A1- 2008 183 077

## Description

### Field of the Invention

The present invention relates to high-intensity focused ultrasound (HIFU) and more particularly to its delivery through a robotic controlled arm at a specified target point for hemostasis and therapeutic purposes.

### Description of Related Art

Ultrasound is a well-established imaging modality in the areas of medicine, biology and engineering. Its ease of application and low cost make it the choice for medical imaging for most soft tissue diagnostic analysis. Ultrasound has been used to examine many parts of the body, including babies in the mother's womb, the vascular system, heart, organs within the abdominal cavity, urinary system, ovaries, brain and most recently, the muscles.

Since the development of ultrasound for therapeutic applications, there have been many advances in the medical use of ultrasound, especially for high intensity focused ultrasound (HIFU). There is great interest in HIFU as a surgical tool due to its non-invasive nature and the ability to focus on the location where therapy occurs. Furthermore, it can be delivered relatively deep within a patient's body without adverse effect on the intervening tissues.

HIFU has been successfully applied in the treatment of cancers, particularly in destroying tumors found in the breast, prostate, kidney and pancreas. Studies and trials include:

Hepatology: Treatment of liver tumors in UK.

Urology: Treatment of benign prostatic hyperplasia (BPH).

Acoustic Hemostasis: Using HIFU to form blood clots or cauterize blood vessels. The inventors view this approach as a potential non-invasive tool for liver trauma and resection.

The effectiveness of HIFU relies heavily on the precision focusing of the transducer at the targeted region, particularly so for multi-probe HIFU. Factors such as the medium between the probe and the contact surface will affect the positioning of the probe. Dynamic change of the intervening tissue properties is another challenge.

Hemostasis is particularly important because bleeding is one of the primary causes of death after traumatic injury. Uncontrolled hemorrhage conditions can be challenging in trauma patients, particularly for those with vascular, splenic, or hepatic injuries. The inability to control bleeding might be due to the malfunction of the organs to produce platelets to stop the bleeding or due to blood diseases. Failure to control hemorrhage can lead to insufficient organ perfusion (shock) and is the major cause of death in trauma victims. Indeed, the major cause of mortality in combat casualties is often reported as exsanguination (bleeding to death). In addition, conducting biopsy procedures onto the organs for diagnoses purposes can also cause the organ to bleed excessively. Therefore there is a need to find ways to solve the problem of uncontrolled internal bleeding especially after biopsy.

Despite the availability of several hemostasis methods such as laser welding and argon beam coagulation, such methods do not arrest the bleeding problem effectively. These existing cauterization methods such as electrocautery and laser can only deliver energy on the surface of tissues with a maximum penetration of 2-3 mm. Hence it is important to find another technique to penetrate energy deeper in the tissues or organs to arrest the bleeding efficiently.

High intensity focused ultrasound (HIFU) therapy is capable of delivering energy deep in the tissue, which makes it a suitable candidate to conduct blood coagulation to stop internal bleeding. Therefore, high intensity focused ultrasound therapy is being investigated as a method for controlling internal hemorrhage. It has been shown that HIFU is quite successful in controlling bleeding in animal trials. For most of the trials, liver is chosen as the organ to perform HIFU hemostasis. The reason is mainly because the liver is vascular, as 25% of the cardiac output flows through it, and significant bleeding can occur. Furthermore, uncontrolled hemorrhage can be fatal.

It has been proposed to perform HIFU hemostasis onto the bleeding spot after the radiologist has conducted liver biopsy. One key reason is that the major complication for liver biopsy is the risk of hemorrhage, and hence a tool is needed to control the hemorrhage. However, HIFU as a tool for controlling hemorrhage is still in its infancy. A number of research centers are still in the process of conducting clinical trials to prove the efficacy of this technique, with most reporting positive results from their experiments.

Another area of development in HIFU is the design and construction of a holder to accommodate and allow smooth delivery of HIFU beams. Some of the factors such as cost, reliability, portability, safety issues and ease of use have to be taken into account during the design process of the holder.

Several structures and devices are designed to accommodate both the ultrasound probe (for imaging purposes) and the HIFU transducer (for therapeutic application). Some of these devices are the Marsden HIFU manipulator and the ULTRABOT. Nanyang Technological University has a long track record for contributing to the development of computer-based technologics to assist in surgery. Some of these researches are the robotic manipulator for prostate treatment and the robotic system for interstitial laser coagulation. In addition, there have also been studies and researches in the area of HIFU for the treatment of cancers. Particularly in the Computer Integrated Medical Laboratory (CIMIL), the focus is on implementing HIFU for the treatment of prostate cancers.

However, accurate aiming of the HIFU transducer relative to the location of the biopsy has not yet been achieved. One particular problem is that once the biopsy has been performed, the hole left by the biopsy is often too small to be detected by medical imaging techniques such as ultrasound, CAT scanning, or MRI.

Document US 5 184 601 describes a surgical system comprising a cart, a cart arm comprising joints equipped with locking mechanisms and a manipulator to which a surgical tool is attached. Document US 2008 18 3077 describes a system designed to stop a hemorrhage with help of HIFU treatment.

### Summary of the Invention

From the above, it will be seen that a need exists in the art for accurate placement of the HIFU transducer relative to the biopsy needle to allow HIFU hemostasis of the specific location of the biopsy.

It is an object of the invention to provide such accurate placement.

It is another object of the invention to provide such accurate placement in a form which can be conveniently used in an operating room or a biopsy room.

It is still another object of the invention to provide such accurate placement in a form which can be at least partially automated.

To achieve the above and other objects, the present invention is directed to a holding structure to hold the biopsy needle and the HIFU transducer to allow the smooth and accurate delivery of the HIFU beam at the interface of the liver (or other organ), where bleeding will occur. The manipulator structure in at least some embodiments performs the biopsy and HIFU through the percutaneous approach. In addition, the procedure can be imaged by the ultrasound imaging probe. The HIFU probe can track the biopsy needle entry site even when the needle has been removed with the help of the robotic arm.. The HIFU transducer a short pulse of HIFU in the needle tract to cause coagulation and stop bleeding. Targeting can be robotically controlled using data extracted from a conventional two-dimensional image taken via any suitable imaging technique (e.g., ultrasound, CAT, or MRI).

In a preferred embodiment, the area in which the biopsy is to be performed is imaged, and coordinates of the location of the biopsy are extracted and input into a processor. The processor robotically controls the manipulator which holds the HIFU probe so that the HIFU probe is focused onto that location. Thus, the above-noted problem of inability to image the hole is overcome.

Other organs for which the present invention is usable include native kidney and transplant kidney in children and adults. The robotically controlled HIFU delivery system can be an independent unit that can be used with computed tomography guided biopsy, magnaetic resonance guided biopsy or ultrasound guided biopsy of the liver, kidney, lung or other organs to control post-biopsy hemorrhage. This unit will be able to control post-biopsy hemorrhage in various organs irrespective of the size of the needle used. This can also be used treating placental disorders such as twin twin transfusion in twin pregnancy and related such diseases. The robotically controlled HIFU system can be used to treat skin disorders such as acne or other skin disorders and diseases.

### Brief Description of the Drawings

A preferred embodiment of the present invention will be set forth in detail with reference to the drawings, in which:

Fig. 1 is a section overview showing the manipulator according to the preferred embodiment;

Fig. 2 shows the main components lined up with the patient;

Fig. 3 shows the sterilized needle holder relative to the ultrasound probe;

Fig. 4 shows the system as applied to the patient;

Fig. 5 shows the manipulator control section;

Fig. 6 shows the system in use;

Fig. 7 shows coagulation patterns; and

Figs. 8 and 9 show a prototype of a system according to the preferred embodiment.

### Detailed Description of the Preferred Embodiment

A preferred embodiment will be described in detail with reference to the drawings, in which like reference numerals refer to like elements or steps throughout.

Shown in Figure I is a section overview of the manipulator assembled onto the arm of the cart. In the system 100, the cart arm 102 supports the manipulator 104 over the body of the patent *P* and specifically over the patient's liver *L*.

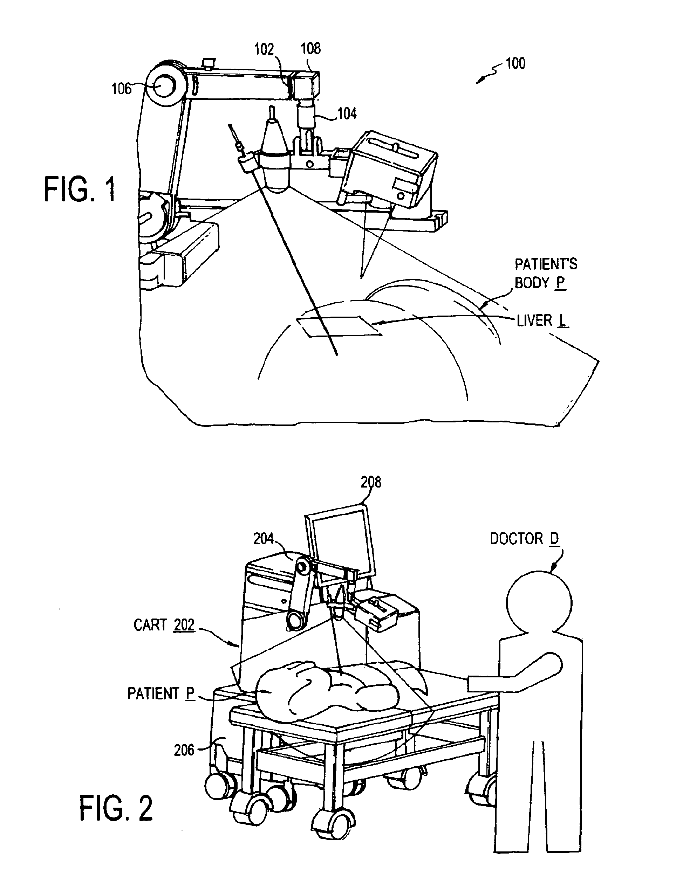

Shown in Figure 2, the main components are lined up with the radiologist *D* beside the patient and facing the screens of the cart 202 and the ultrasound scanner 204 which is positioned on the right of the cart. The cart includes a base portion having a processor 206 for performing all of the processing disclosed herein, including automatic control, as well as a touch screen 208 for displaying images to the operator and for receiving inputs from the operator.

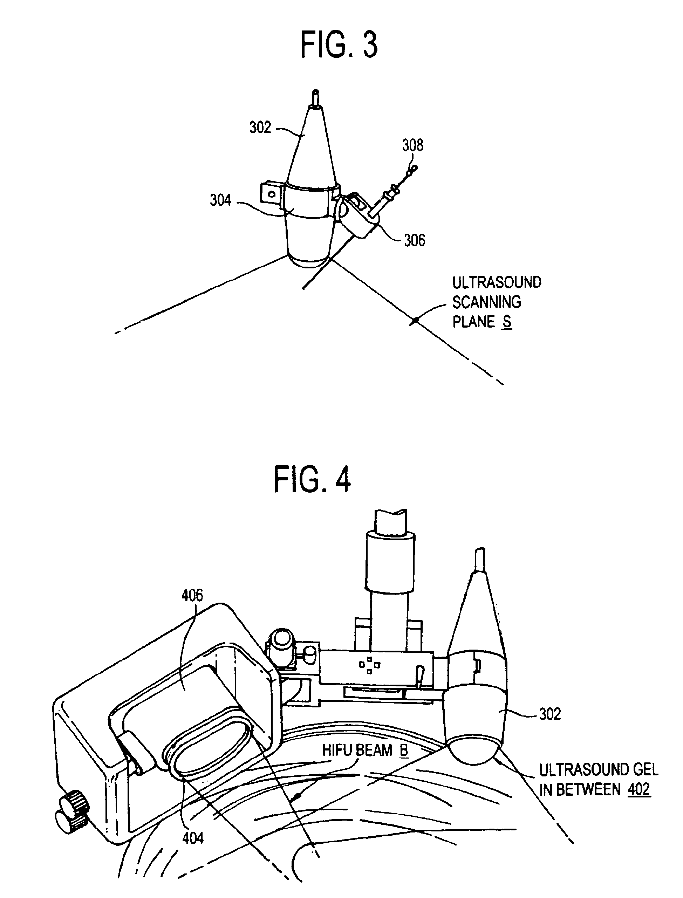

The sterilized needle holder is self aligned to the ultrasound probe holder as shown in Figure 3. More specifically, the ultrasound probe holder 302 is held by an ultrasound probe holder 304, which also supports a needle holder 306 holding a biopsy needle 308. The ultrasound probe will be used to emit ultrasound waves in an ultrasound scanning plane S.

A layer of ultrasound gel 402 is applied to the patient's skin and a water bag 404 attached to the HIFU transducer 406 as shown in Figure 4. The water level in the water bag is automatically determined by the pressure sensor beside the HIFU transducer. The HIFU transducer will be used to emit a HIFU beam *B*.

The radiologist performs an ultrasound scan to locate the area of operation by looking at the screen of the ultrasound scanner. The scan is done freehand by the radiologist. In this step, only the ultrasound probe and the biopsy needle which are mounted on their respective holders and assembled together as shown in Figure 3 are used.

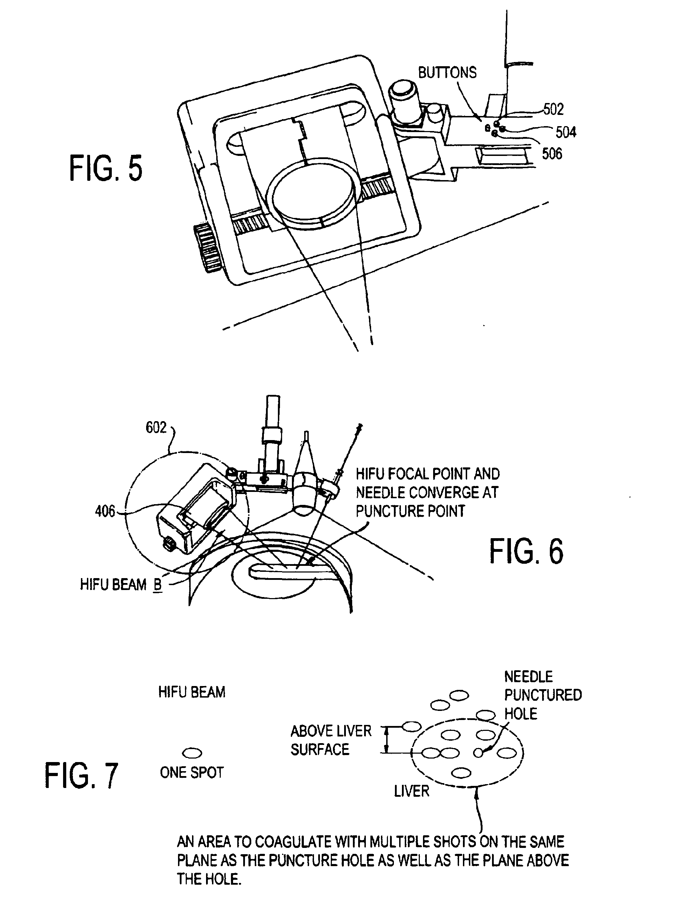

Upon locating the target point, the radiologist brings in the manipulator which is assembled to the cart arm and combined with the ultrasound probe holder. The manipulator is now complete as seen in Figure 1. Both the components: the HIFU transducer and the biopsy needle are contained in the 2D-ultrasound plane of the ultrasound probe at all times throughout the operation. The cart arm and the manipulator are both frozen in their position by magnetic locks shown in Figure 1 as 106 and 108. They can be freed by pressing the light blue button 502 on the center piece of the manipulator as shown in Figure 5 while moving both the components to the desired position. The components are frozen in their position once the button is released. Likewise for fine adjustments or just the manipulator, the dark blue button 504 as indicated in Figure 5 can be depressed to free the manipulator and freeze the manipulator when released.

After the final adjustments are done, the radiologist injects the biopsy needle into the liver and commences the operation. Upon completion, just before the radiologist removes the needle, the automated section 602 including the HIFU transducer is moved automatically to the target area of coagulation which is the punctured surface of the liver via the mechanisms as shown in Figure 6 by using the touch screen on the cart. The coagulation can be done over an area or volume circling the needle entry point as shown in Figure 7 by selecting computer default programmed schemes from the touch screen or selecting pre-programmed coagulation patterns prepared by the radiologist before the commencement of the operation. Once the biopsy needle is removed, the radiologist presses the red button 506 once as indicated in Figure 5 to trigger the HIFU transducer which carries out the coagulation on the punctured point of the liver surface as specified by the radiologist via the touch screen.

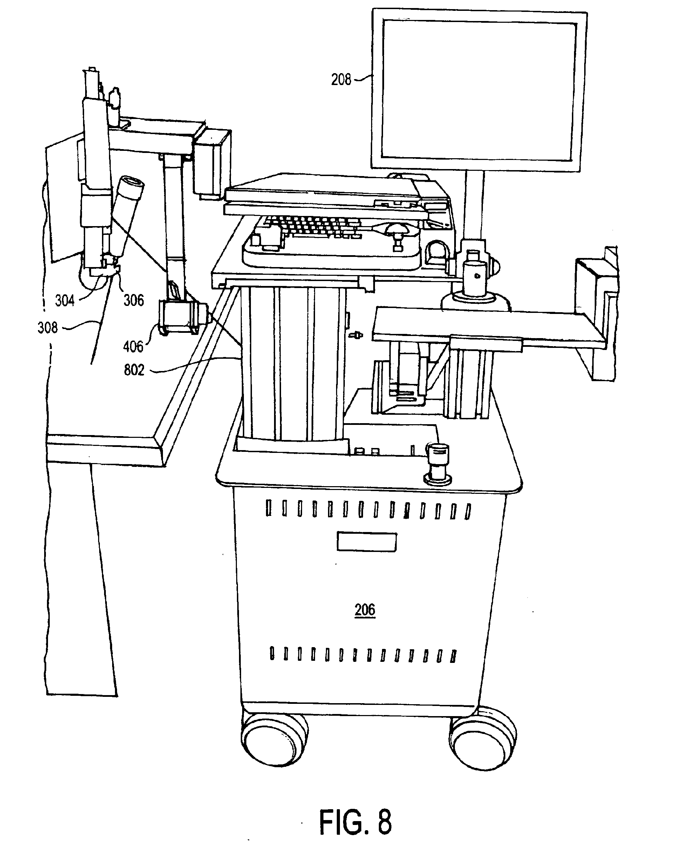

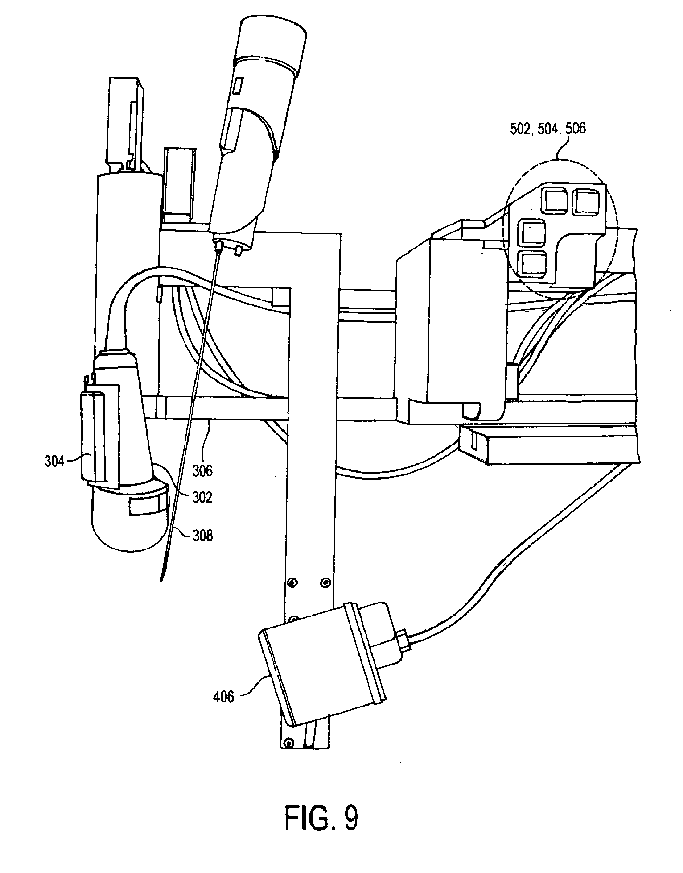

Figs. 8 and 9 show a prototype. In addition to the features described above, the prototype includes a hydraulic lift section 802.

While a preferred embodiment has been set forth in detail above, those skilled in the art who have reviewed the present disclosure will readily appreciate that other embodiments can be realized within the scope of the invention. For example, the invention is not limited to use with the liver, but can instead be used to perform biopsies on any organ. Also, instead of or in addition to the disclosed buttons and touch screen, any suitable user interface can be used.

Therefore, the present invention should be construed as limited only by the appended claims.

## Claims

1. A system (100) for performing a biopsy on an organ and performing high-intensity focused ultrasound on the organ to stop bleeding at a location of the biopsy,
the system comprising:
a cart (202);
a cart arm (102) on the cart, the cart arm comprising a first locking mechanism (106) such that when the first locking mechanism is unlocked, the cart arm is movable relative to the cart, and when the first locking mechanism is locked, the cart arm is fixed in place relative to the cart;
a manipulator (104) on the cart arm, the manipulator comprising a second locking mechanism (108) such that when the second locking mechanism is unlocked, the manipulator is movable relative to the cart arm, and when the second locking mechanism is locked, the manipulator is fixed in place relative to the cart arm; **characterised by** :
a biopsy needle (308) on the manipulator; and
a high-intensity ultrasound transducer (406) on the manipulator.

2. The system of claim 1, further comprising an imaging ultrasound transducer (302) attached to the manipulator.

3. The system of claim 2, wherein the biopsy needle (308) is attached to the imaging ultrasound transducer (302).

4. The system of claim 2, further comprising:
a processor (206), in communication with the imaging ultrasound transducer, programmed to produce an ultrasound image in accordance with ultrasound signals received from the imaging ultrasound transducer; and
a display (208) for displaying the ultrasound image to the operator.

5. The system of claim 4, wherein the processor is further programmed to locate in the ultrasound image a location in the organ in which the biopsy is performed and
to control the manipulator to move such that the high-intensity focused ultrasound transducer is positioned to emit a high-intensity focused ultrasound beam which is focused on the location in the organ in which the biopsy has been performed.

6. The system of claim 4, further comprising a first control (502) for actuation by the operator to unlock the first locking mechanism and the second locking mechanism.

7. The system of claim 6, further comprising a second control (504) for actuation by the operator to unlock only the second locking mechanism.

8. A system (100) for performing a biopsy on an organ and performing high-intensity focused ultrasound on the organ to stop bleeding at a location of the biopsy, the system comprising:
a robotically controlled manipulator (104);
an imaging device (302) for taking an image of the organ; **characterised by** a biopsy needle (308) on the manipulator;
a high-intensity ultrasound transducer (406) on the manipulator;
and a processor (206) programmed to control the manipulator to focus the high-intensity ultrasound transducer to focus on the location of the biopsy and to emit highfrequency ultrasound to stop the bleeding.

9. The system of claim 8, wherein the imaging device comprises an ultrasound imaging device.

## Patentansprüche

1. System (100) zur Durchführung einer Biopsie an einem Organ und zur Anwendung von hochintensivem fokussiertem Ultraschall an dem Organ zur Stillung einer Blutung an einer Stelle der Biopsie, wobei das System umfasst:
einen Gerätewagen (202),
einen Gerätewagenarm (102) an dem Gerätewagen, wobei der Gerätewagenarm einen ersten Verriegelungsmechanismus (106) umfasst, so dass der Gerätewagenarm bei entriegeltem erstem Verriegelungsmechanismus relativ zum Gerätewagen beweglich ist und bei verriegeltem erstem Verriegelungsmechanismus relativ zum Gerätewagen fixiert ist,
einen Manipulator (104) am Gerätewagenarm, wobei der Manipulator einen zweiten Verriegelungsmechanismus (108) umfasst, so dass der Manipulator bei entriegeltem zweitem Verriegelungsmechanismus relativ zum Gerätewagenarm beweglich ist und der Manipulator bei verriegeltem zweitem Verriegelungsmechanismus relativ zum Gerätewagenarm fixiert ist,
**gekennzeichnet durch**
eine Biopsienadel (308) an dem Manipulator und
einen hochintensiven Ultraschall-Wandler (406) an dem Manipulator.

2. System nach Anspruch 1, darüber hinaus umfassend einen an dem Manipulator angebrachten bildgebenden Ultraschallwandler (302).

3. System nach Anspruch 2, wobei die Biopsienadel (308) an dem bildgebenden Ultraschallwandler (302) angebracht ist.

4. System nach Anspruch 2, darüber hinaus umfassend:
einen Prozessor (206) in Kommunikationsverbindung mit dem bildgebenden Ultraschallwandler, programmiert zur Erstellung eines Ultraschallbildes anhand der von dem bildgebenden Ultraschallwandler erhaltenen Ultraschallsignale, und
ein Display (208), um dem Betreiber das Ultraschallbild anzuzeigen.

5. System nach Anspruch 4, wobei der Prozessor darüber hinaus programmiert ist, im Ultraschallbild eine Stelle in dem Organ, in dem die Biopsie durchgeführt wird, zu lokalisieren und den Manipulator entsprechend zu steuern, dass er sich so bewegt, dass der hochintensive fokussierte Ultraschall-Wandler positioniert wird, um einen hochintensiven fokussierten Ultraschallstrahl abzustrahlen, der auf die Stelle in dem Organ, in dem die Biopsie durchgeführt wurde, fokussiert ist.

6. System nach Anspruch 4, darüber hinaus umfassend eine erste Steuerung (502) zur Betätigung durch den Betreiber, um den ersten Verriegelungsmechanismus und den zweiten Verriegelungsmechanismus zu entriegeln.

7. System nach Anspruch 6, darüber hinaus umfassend eine zweite Steuerung (504) zur Betätigung durch den Betreiber, um nur den zweiten Verriegelungsmechanismus zu entriegeln.

8. System (100) zur Durchführung einer Biopsie an einem Organ und zur Anwendung von hochintensivem fokussiertem Ultraschall an dem Organ zur Stillung einer Blutung an einer Stelle der Biopsie, wobei das System umfasst:
einen Roboter-gesteuerten Manipulator (104),
eine bildgebende Vorrichtung (302) zur Aufnahme eines Bildes des Organs,
**gekennzeichnet durch**
eine Biopsienadel (308) an dem Manipulator,
einen hochintensiven Ultraschall-Wandler (406) an dem Manipulator und
einen Prozessor (206), der zur Steuerung des Manipulators programmiert ist, so dass dieser den hochintensiven Ultraschall-Wandler fokussiert, um ihn auf die Stelle der Biopsie zu fokussieren und hochfrequenten Ultraschall abzustrahlen, um die Blutung zu stillen.

9. System nach Anspruch 8, wobei die bildgebende Vorrichtung eine Ultraschallbildgebungsvorrichtung aufweist.

## Revendications

1. Système (100) pour réaliser une biopsie sur un organe et actionner des ultrasons focalisés à haute intensité sur l'organe, afin d'arrêter le saignement à l'emplacement de la biopsie, le système comprenant :
un chariot (202) ;
un bras de chariot (102) sur le chariot, ledit bras de chariot comprenant un premier mécanisme de verrouillage (106), de sorte que, lorsque le premier mécanisme de verrouillage est déverrouillé, le bras de chariot soit mobile relativement au chariot, et
lorsque le premier mécanisme de verrouillage est verrouillé, le bras de chariot soit fixé en place relativement au chariot ;
un manipulateur (104) sur le bras de chariot, le manipulateur comprenant un second mécanisme de verrouillage (108), de sorte que, lorsque le second mécanisme de verrouillage est déverrouillé, le manipulateur soit mobile relativement au bras de chariot, et lorsque le second mécanisme de verrouillage est verrouillé, le manipulateur soit fixé en place relativement au bras de chariot ; **caractérisé par** une aiguille de biopsie (308) sur le manipulateur ; et
un transducteur à ultrasons haute intensité (406) sur le manipulateur.

2. Système selon la revendication 1, comprenant en outre un transducteur à ultrasons d'imagerie (302) fixé au manipulateur.

3. Système selon la revendication 2, dans lequel l'aiguille de biopsie (308) est fixée au transducteur à ultrasons d'imagerie (302).

4. Système selon la revendication 2, comprenant en outre :
un processeur (206), en communication avec le transducteur à ultrasons d'imagerie,
programmé pour produire une image en ultrasons conformément à des signaux d'ultrasons reçus du transducteur à ultrasons d'imagerie, et
un écran (208) pour afficher l'image en ultrasons à l'opérateur.

5. Système selon la revendication 4, dans lequel le processeur est en outre programmé pour localiser sur l'image en ultrasons un emplacement dans l'organe dans lequel la biopsie est réalisée et pour contrôler le manipulateur afin qu'il se déplace pour que le transducteur à ultrasons focalisés à haute densité soit positionné de façon à émettre un faisceau d'ultrasons focalisés à haute densité qui est focalisé sur l'emplacement dans l'organe où la biopsie a été réalisée.

6. Système selon la revendication 4, comprenant en outre une première commande (502) permettant l'actionnement par l'opérateur, afin de déverrouiller le premier mécanisme de verrouillage et le second mécanisme de verrouillage.

7. Système selon la revendication 6, comprenant en outre une seconde commande (504) pour permettre l'actionnement par l'opérateur, afin de déverrouiller uniquement le second mécanisme de verrouillage.

8. Système (100) pour réaliser une biopsie sur un organe et actionner des ultrasons focalisés à haute intensité sur l'organe, afin d'arrêter le saignement à l'emplacement de la biopsie, le système comprenant :
un manipulateur contrôlé par robot (104) ;
un dispositif d'imagerie (302) pour prendre une image de l'organe ; **caractérisé par** une aiguille de biopsie (308) sur le manipulateur ;
un transducteur à ultrasons haute intensité (406) sur le manipulateur ;
et un processeur (206) programmé pour commander le manipulateur afin de focaliser le transducteur à ultrasons haute intensité sur l'emplacement de la biopsie et
d'émettre des ultrasons à haute fréquence pour arrêter le saignement.

9. Système selon la revendication 8, dans lequel le dispositif d'imagerie comprend un dispositif d'imagerie à ultrasons.
